# EUROPEAN PATENT APPLICATION

(11) **EP 1 776 978 A1**
(43) Date of publication of application: **25.04.2007**
(21) Application number: 06122638.7
(22) Date of filing: 20.10.2006
(51) Int. Cl.: A61M 16/00, A61M 5/00

(54) **System for delivering anesthesia drugs to a patient**

(30) Priority: 21.10.2005 EP 05109830
(71) Applicant: GENERAL ELECTRIC COMPANY, Schenectady, NY 12345 (US)
(72) Inventor: Uutela, Kimmo, 00100 Helsinki (FI); Rantala, Börje, 00670 Helsinki (FI); Pesu, Leena, 00810 Helsinki (FI); Yli-Hankala, Arvi, 33270 Tampere (FI)
(74) Representative: Valkeiskangas, Tapio Lassi Paavali

(57) **Abstract**

The invention relates to a system for delivering anesthesia drugs to a patient, the system comprising means (1) for feeding anesthesia drugs to the patient treated, a measuring device (2) for measuring at least one parameter relating to the effects of the anesthetic drugs fed to the patient, a patient monitor (3) to show the results measured by the measuring devices, and feeding means for feeding patient demographics to the system. The system further comprises means (5) for anesthesia management for drug effect during the treatment, the means for anesthesia management. The means for anesthesia management (5) is connected to a control algorithm that links the parameter values to the drug delivery changes.

## Description

The invention relates to a system for delivering anesthesia drugs to a patient, the system comprising means for feeding anesthesia drugs to the patient treated, a measuring device for measuring at least one parameter relating to the effects of the anesthetic drugs fed to the patient, a patient monitor to show the results measured by the measuring devices, and feeding means for feeding patient demographics to the system.

Anesthesia is typically given (approximately 90% of the normal day surgery) by both intravenous and inhalational gas anesthetics. There exits however certain amount of users that practice total intravenous anesthesia (TIVA) especially in Europe and more widely TIVA can be used in small & fast cases. During the anesthesia anesthesiologist titrates the anesthetics based on drug models and the drug distribution over time as well as by following the physiological parameters (the effect or responses to the drug given).

Intravenous (IV) drug distribution and model behaviour can be followed sometimes from an intravenous pump (IV pump) display (there exists some devices on the market, called TCI pumps) but in most of the cases there is no modelling or distribution shown. In practice the anesthesiologist still calculates an appropriate amount of drug for the particular patient with a calculator or in his head (the inputs being weight and height). This happens with the commonly used syringe pumps having no models inside them. Also the pumps are many times away from the parameter display (patient monitor) and so the effects and responses to the drugs are shown separately from the IV drug management.

The gas anesthetic statistical model called MAC (minimal alveolar concentration) is shown in most anesthesia monitors. This is however a quite approximate measure, since its background is on tests where the endpoint has been skin incision (and not for example the true hypnosis level). This measure is shown in some integrated anesthesia machines in the parameter display (patient monitor), so the anesthesiologist can see the drug effect and response quite easily. Another measure of gas anesthetics effect is the end tidal agent (EY) or fraction of inspired agent (FI), which can be shown in anesthesia monitor.

The first step to integrate the IV drug models into one display has been taken by GE Healthcare by launching a Care Assistant Suite (CAS) in European Anesthesiologist Congress 2005. This display shows IV drug models and their synergetic effects in one display, which is mounted next to the patient monitor and the anesthesia machine.

During the years several systems and methods have been developed in order to facilitate anesthesia drug deliveries. As typical examples of the solutions of the prior art the following publications can be mentioned.

PCT Publication W00232036A2 describes a method and an apparatus for monitoring anesthesia drug dosages, concentration, and effects using N-dimensional representations of critical functions. This patent describes a method, system and apparatus for the monitoring, diagnosis and evaluation of the state of a dynamic system. The method and system provides the processing means for receiving sensed and/or simulated data, converting such data into a displayable object format and displaying such objects in a manner such that the interrelationships between the respective variables can be correlated and identified by a user. In particular, this invention is directed to the processing and display of drug data for the use of doctors in the process of monitoring or administering drugs to patients.

US patent application 2003/0051737 A1 describes an apparatus and a method for titrating drug delivery. In this application a method and apparatus for reducing the workload of titrating drug (sedative, amnesic and & or analgesic) to effect while leaving clinician users in control of a related procedure is described. A drug delivery device is controlled to achieve a target drug concentration at a selected site in the patient or a predetermined infusion rate waveform. The time profile of the target drug concentration or a predetermined infusion rate waveform is controlled by a drug state model that uses clinical heuristics to implement safe, pre-defined changes in the target drug concentration or infusion rate and user-commanded changes in target drug concentration or infusion rate.

US patent application 2002/0169636 A1 describes a system and method for managing patient care. The application is directed to a system and method for providing care to a patient, comprising a patient care device having a number of configuration databases stored in a memory of the device. Each configuration database preferably includes protocols, operating limits, rule sets and/or operating features that collectively define an operating environment, or personality, of the device. The selected protocol includes default parameters for delivering the drug, and the label optionally includes instructions for deviating from the default protocol.

US Patent 6 631 291 describes a closed loop drug administration method and apparatus using EEG complexity for control purposes. A closed loop method and apparatus for controlling the administration of a hypnotic drug to a patient. At least one measure of the complexity of the EEG signal data is derived from the patient. An EEG signal complexity measure obtained from the cerebral activity of the patient can be advantageously used in conjunction with a measure of patient electromyographic (EMG) activity to improve the response time of hypnotic level determination and of the feedback control of drug administration. A pharmacological transfer function may be used, along with pharmacokinetic and pharmacodynamic models.

EP Patent Application 1 547 631 A1 describes a computer-controlled intravenous drug delivery system. The system described relates to controlling and steering intravenous anesthesia (IVA) and/or the application of other intravenous drugs to a patient in a safe and user friendly way. Less experienced anesthetists profit from expert knowledge stored, retrievable and usable via the system.

The systems and methods known in the prior art are however rather complicate and not so user friendly and flexible in every day life.

The object of the invention is to obtain a system with which the disadvantages of the prior art can be eliminated. This is obtained with the invention. The invention offers an enhanced way to do anesthesia and simplifies the procedures to be carried out in certain circumstances. Standard adjustments can be easily carried out and the user is well aware of the effect of the changes. As an example of the circumstances in which the invention is especially advantageous time-critical procedures can be mentioned, i.e. time-critical procedures can be automatized and therefore eventual delays in critical situations can be eliminated. The invention is related especially, but not exclusively to balanced anesthesia containing intravenous and inhalational gas anesthetics. The invention is characterized in that the system comprises means for anesthesia management for drug effect during the treatment, the means for anesthesia management being connected to a control algorithm that links the parameter values to the drug delivery changes.

The advantage of the invention is its simplicity and flexibility. In other words the invention can be easily used with the known systems, for example the CAS display as described above. The invention can also be used in connection with patient monitors or anesthesia machines if needed. In other words the invention can be advantageously adapted to the various devices already existing for example in a hospital, and therefore investment costs are relatively low.

In the following the invention will be described in greater detail by means of examples described in the attached drawings, in which
Figure 1 shows a block diagram of the invention,
Figure 2 shows a block diagram of one possible example of the system shown in Figure 1,
Figure 3 shows a block diagram of another possible example of the system shown in Figure 1,
Figure 4 shows one possible embodiment of the user interface for the system shown in Figure 1,
Figure 5 shows another possible embodiment of the user interface for the system shown in Figure 1, and
Figure 6 shows one possible detail of the user interface for the system shown in Figure 1.

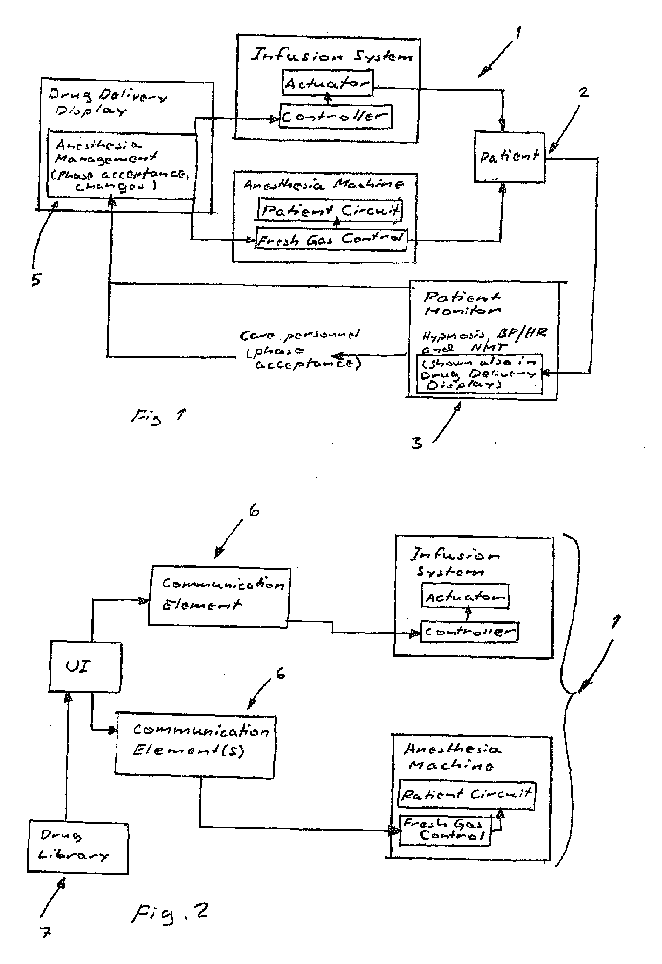

Figure 1 shows a block diagram of the invention. The term anesthesia management refers here to proceedings carried out when the actual drug deliver is happening. The effects of the drugs can be seen in the display and the user can change the drug delivery based on the parameter information and the surgery changes. The phase timing information would also come from the user by acceptance of the next phase. This way the next phase drug delivery can take place only after the previous phase is over.

The user interface of the anesthesia management can be based on modelled drug effects PK (pharmacokinetic) and/or PD (pharmacodynamic) for IVs and MAC or ET or FI for gas anesthetics. Otherwise it can be based on parameters like hypnotic measure, analgesic measure and NMT (neuromuscular transmission) measure. Said proceedings are known per se in the field. This could be connected to a control algorithm that links the parameter values to the drug delivery changes.

The display could as well bring advisories and alarms based on the measured patient values or other clinical practices that would need to be taken into account during the case. There could be user set alarm limits and other safety features, for example quick hypnotic as will be described later.

The invention can also be used together with anesthesia planning as described in EP Patent Application 05 109 830.9. As described in said EP Patent Application the anesthesia planning can be based on modelled drug effects PK and/or PD for IVs and MAC or ET or FI for gas anesthetics, and the parameter information could be shown aside. Otherwise it can be based on parameters like hypnotic measure, analgesic measure and NMT measure. The User Interface could be used also so that the parameter values would have user set limits and there would come advisories or alarms when the limits are approaching, so that the user himself or herself could manage the drug deliveries. The phase timing information could also come from the user by acceptance of the phases.

As told above the invention is related to anesthesia control especially in balanced anesthesia containing intravenous (IV) and inhalational gas anesthetics. It should however be noted that the invention can also quite well be used in connection with intravenous anesthetics only or respectively with inhalational anesthetics only. The invention can also combine visual anesthesia planning done before the case with the anesthesia management during the anesthesia. The system brings to the user an easier way to manage different anesthesia phases by giving a user interface where both the IVs and gas anesthetics can be managed from the same display. If also anesthesia planning is used, the anesthesia planning gives the possibility for the clinic to spread the best practices by using the predefined anesthesia templates for different anesthesia cases. The phases can be visualized for example on the basis of drug modelling for intravenous anesthetics, gas anesthetics or end tidal concentration.

The system of the invention comprises means 1 for feeding anesthesia drugs to the patient treated. The means 1 for feeding anesthesia drugs can comprise infusion system or/and anesthesia machine. The system comprises further measuring devices 2 for measuring parameters relating to the effects of the anesthetic drugs fed to the patient, and a patient monitor 3 to show the results measured by the measuring devices. The measuring device 2 can be. For example, modelled drug effects based on the amount of IV drugs given to the patient, the measured ET or FI concentration of gas anesthetics, EEG-based estimate of the hypnotic measure of the patient, measure of the analgesic effect based on the stress-related physiological changes, or NMT measure of paralysis. The system comprises also feeding means for feeding patient demographics to the system. The term patient demographics refer to patient information such as age, sex, height, weight etc. Said means can comprise appropriate equipment for manual feeding or/and said means can be connected to an information management system available. The information management system can be adapted also to feed all kinds of other important information to the system, for example eventual drug related inconsistencies such as allergies, diseases, previous drug delivery related problems etc. The essential idea of the invention is that the system further comprises means 5 for anesthesia management for drug interaction during the treatment. Some advisories and/or alarms could come from the patient monitor 3 interface that is connected to the drug delivery display. The means 5 and patient monitor 3 are shown in figure 1.

As described before the invention described in Figure 1 can also be combined with anesthesia planning, i.e. drug delivery display shown in Figure 1 can also comprise means for visual planning of the anesthesia drug delivery before the drugs are actually fed.

The system shown in Figure 1 requires algorithm that links the parameter values, i.e. values obtained as a result from measurements from the patient, to the drug delivery changes. The anesthesia management system allows also the user to change the drug delivery according to the parameter information and surgery changes. The phase timing information can also here come from the user by acceptance of the next phase by using an appropriate mechanism for example through a touch screen, mouse or remote controller.

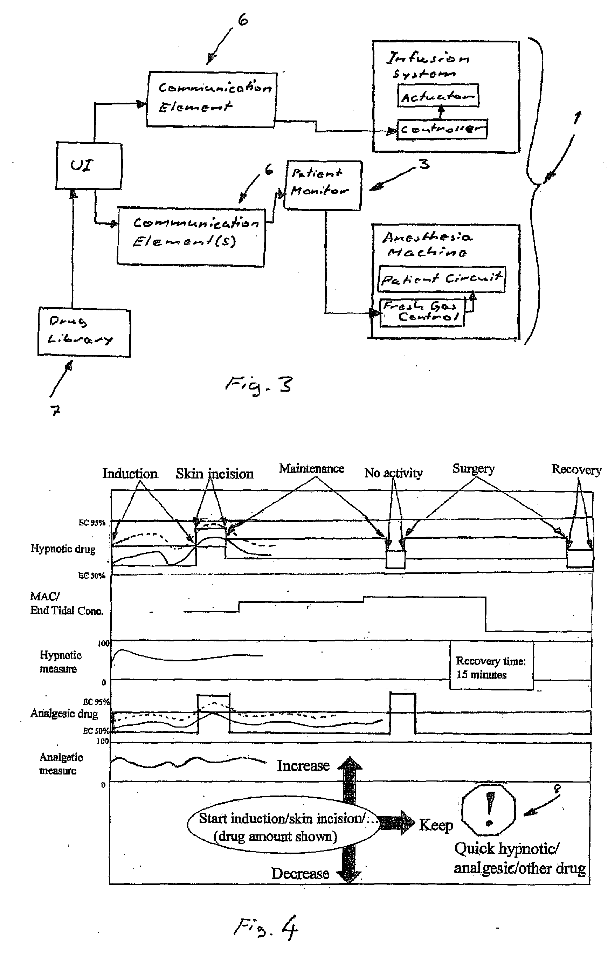

As described above if the invention is seen on the upper level the different elements of the invention are those shown in Figure 1. Figure 1 shows clearly that on this level the main elements in the system are means 1 for feeding anesthesia drugs, i.e. the infusion system and/or anesthesia machine, the user interface, i.e. means 5 for anesthesia management and communication elements 6 with which the user interface can communicate with means 5. The basic idea in the invention is that the system comprises means 5 for anesthesia management for drug effect during the treatment, and that the means for anesthesia management 5 is connected to a control algorithm that links the parameter values to the drug delivery changes. Said drug effect can also comprise drug interaction, i.e. the action resulted for example from the combined effect of two or more drugs. Some embodiments of said communication elements are described more clearly in figures 2 and 3. In the diagrams of figures 2 and 3 block Ul refers to the user interface comprising for example means 5 shown in figure 1.

As shown in figure 2 the communication elements could be constructed from a software including drivers of the devices, networked server or similar. The communication elements could be part of the UI device as well. The UI device shall include watchdog or equivalent as a safety feature. The infusion system could be either a syringe pump or a TCI pump when the drug library 7 could be in the pump instead of a separate server. In the IV pump there would be the controller and actuator to handle the drug titration. The anesthesia machine would contain the fresh gas control unit (to do the actual drug titration) and the patient circuit to deliver the drugs to the patient. The patient monitor would provide the parameter information to the users and to the Drug Delivery Display. The anesthesia machine would be left out in TIVA cases.

Another possibility is that the Ul communicates with the anesthesia machine 3 through the patient monitor 3. This embodiment is shown in figure 3.

The user interface of the anesthesia management 5 can be based on modelled drug effects PK and/or PD for IVs and MAC or ET or FI for gas anesthetics as shown in figure 4. Otherwise it can be based on parameters like hypnotic measure, analgesic measure and NMT measure as shown in figure 5. This could be connected to a control algorithm that links the parameter values to the drug delivery changes. The User Interface shown in figures 4 and 5 could be used also so that the parameter values would have user set limits and there would come advisories or alarms when the limits are approaching, so that the user could by himself or herself manage the drug deliveries.

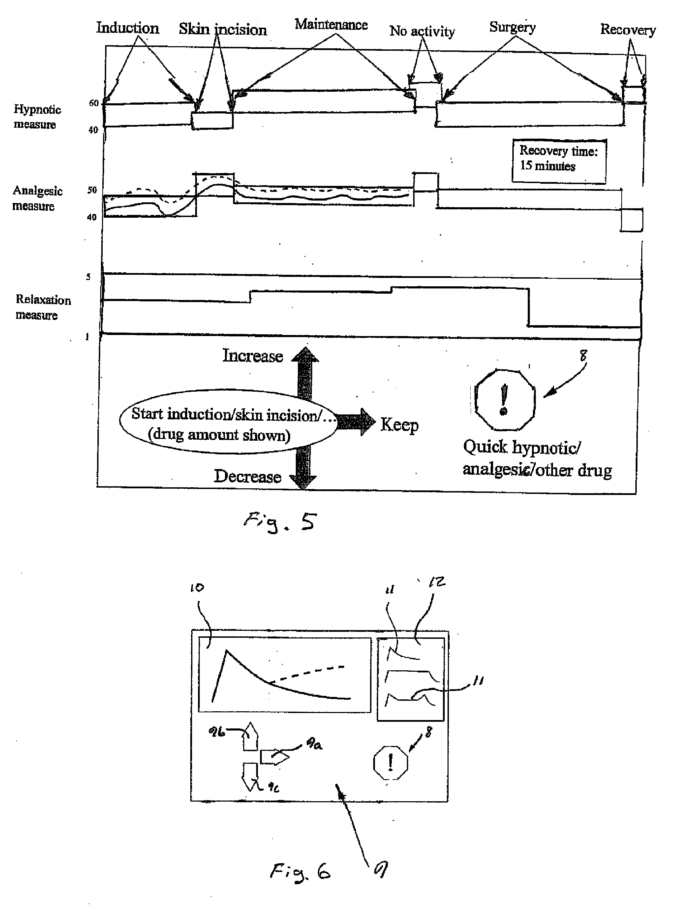

The display could as well bring advisories and alarms based on the measured patient values or other clinical practices that would need to be taken into account during the case. There could be user set alarm limits and other safety features, for example quick hypnotic 8, i.e. the means 5 for anesthesia management comprises a user interface having control means for selecting appropriate preconfigured control algorithm that leads to a desired result. Said control means can be for example a switch arrangement 9 comprising appropriate means, for example a press button 9a or the like, for keeping the patient with the same measured anesthesia level or drug concentration. Said switch arrangement is shown in Figure 6. The purpose of the press button 9a is for example to adjust the level of anesthesia in relation to the current observed state of the patient. When appropriate level has been reached, the press button 9a is pressed and the system keeps the patient with the same measured anesthesia level or drug concentration.

The switch arrangement 9 comprises further means 9b for slightly increasing the anesthesia level or drug concentration. The switch arrangement 9 shown comprises further means 9c for slightly decreasing the anesthesia level or drug concentration. Said increasing and decreasing takes place in pre-determined manner when the means 9b, 9c are used. Said means 9b, 9c can be for example press buttons. The increase or decrease must be used when slight increases or decreases must be applied. The effect on the patient is similar regardless of the type of anesthetic drugs used.

The switch arrangement 9 discussed above can also comprise means for automatically selecting the procedure, which leads to quick increase in hypnotic drug concentration. Said means is shown in Figure 6 with a reference number 8. The means 8 can be for example a press button. The procedure leading to quick increase in hypnotic drug concentration takes place in a pre-determined manner when the press button 8 is pressed.

If the patient becomes aware during operation, he or she may not explicitly recall that if he or she is immediately re-anesthetized. However, after an unexpected arousal, deciding what to do may cause too long a delay. The purpose of quick hypnotic press button 8 is to automatically select the procedure that leads to quick increase in hypnotic drug concentration. For IV drugs, this typically means a bolus dose of correct size. For volatile anesthetic both an increase in drug concentration and minute volume are needed. The correct dose depends on the size of the subject and current drug levels, but may also depend on the length of the remaining operation or the measured sensitivity of the patient.

The user interface can also comprise a predictive graphical display 10 for showing the predicted effects of the procedure selected by the switch arrangement discussed above. The predictive graphical display is used to clarify the effect of the changes made. For example when the quick hypnotic button 8 is pressed, the display shows the predicted hypnosis or drug concentration of the subject, with current and suggested protocol. The user can thus check the maximum drug level and the minimum time of waking up before confirming the change. The graphical display can also be used directly to select the desired levels of hypnosis or analgesia during different phases of operation.

The system shown may also comprise a library of preconfigured templates 11 that can be used to select correct rate of changes. The templates 11 can be shown in the graphical display 12. For example, when the operation end is approaching, the user can select a fast or smooth version of recovery from anesthesia.

The embodiments described above are by no means intended to restrict the invention, but the invention may be modified completely freely within the scope of the claims. Thus it is obvious that the details need not be exactly identical with those shown in the figures and described in the text, but other solutions are also possible within the spirit of the invention.

## Claims

1. System for delivering anesthesia drugs to a patient, the system comprising means (1) for feeding anesthesia drugs to the patient treated, a measuring device (2) for measuring at least one parameter relating to the effects of the anesthetic drugs fed to the patient, a patient monitor (3) to show the results measured by the measuring devices, and feeding means for feeding patient demographics to the system, **characterized in that** the system comprises means (5) for anesthesia management for drug effect during the treatment, the means for anesthesia management (5) being connected to a control algorithm that links the parameter values to the drug delivery changes.

2. The system of claim 1, **characterized in that** the means (5) for anesthesia management comprises a user interface having control means for selecting appropriate preconfigured control algorithm that leads to a desired result.

3. The system of claim 2, **chracterized in that** the control means is a switch arrangement (9) comprising means (9a) for keeping the patient with the same measured anesthesia level or drug concentration.

4. The system of claim 3, **characterized in that** the switch arrangement (9) further comprises means (9b) for slightly increasing, in predetermined manner, the anesthesia level or drug concentration.

5. The system of claim 3, **characterized in that** the switch arrangement (9) further comprises means (9c) for slightly decreasing, in predetermined manner, the anesthesia level or drug concentration.

6. The system of claim 3, **characterized in that** the switch arrangement (9) comprises means (8) for automatically selecting the procedure which leads to quick increase, in pre-determined manner, in hypnotic drug concentration.

7. The system of any of the claims 3-6, **characterized in that** the user interface comprises a predictive graphical display (10) for showing the predicted effects of the procedure selected by the switch arrangement (9).

8. The system of any of claim 1, **characterized in that** the means for anesthesia management comprises a library of preconfigured templates (11) for selecting the correct rate of changes.

9. The system of claim 8, **characterized in that** the templates (11) are shown in the graphical display (12).

10. The system of claim 1, **characterized in that** the means for feeding anesthesia drugs comprise means for inhalational gas anesthetics and/or intravenous anesthetics.

11. The system of claim 2, **characterized in that** the user interface shows different phases of the drug feeding action.

12. The system of claim 11, **characterized in that** the different phase are timed by care personnel.

13. The system of claim 7, **characterized in that** the predictive graphical display (10) shows also parameters measured from the patient.

14. The system of claim 2, **characterized in that** the interface is visualized on the basis of modelled drug effects PK and/or PD for intravenous anesthetics and MAC or ET or FI for gas anesthetics.

15. The system of claim 6, **characterized in that** rate of volatile hypnotic drugs given to the patient is increased by increasing anesthetic concentration in the gas and by increasing the flow of gas.

16. The system of claim 1, **characterized in that** the drug effect comprises drug interaction.
